# EUROPÄISCHE PATENTSCHRIFT

(11) **EP 1 997 423 B1**
(45) Veröffentlichungstag und Bekanntmachung des Hinweises auf die Patenterteilung: **06.07.2011**
(21) Anmeldenummer: 08156255.5
(22) Anmeldetag: 15.05.2008
(51) Int. Cl.: A61B 3/135, A61F 9/007, G02B 21/00, G02B 21/08

(54) **Operationsmikroskop mit Beleuchtungseinrichtung**
Operation microscope with illumination device
Microscope d'opération doté d'un dispositif d'éclairage

(30) Priorität: 31.05.2007 DE 102007025606; 29.08.2007 DE 102007041003
(43) Veröffentlichungstag der Anmeldung: 03.12.2008
(73) Patentinhaber: Carl Zeiss Surgical GmbH, 73447 Oberkochen (DE)
(72) Erfinder: Abele, Alfons, 73527, Schwäbisch Gmünd (DE); Reimer, Peter, 73479, Ellwangen (DE); Seiwert, Anja, 73431 Aalen (DE); Strähle, Fritz, 73540, Heubach (DE); Merz, Franz, 73433, Aalen (DE)
(74) Vertreter: Gauss, Nikolai

(56) Entgegenhaltungen:
- EP-A- 1 109 046
- EP-A- 1 410 754
- DE-A1- 10 304 267
- DE-A1- 19 611 044
- US-B1- 6 236 502

## Beschreibung

Die Erfindung betrifft ein Operationsmikroskop mit einer Beleuchtungseinrichtung, die mit einem ersten Beleuchtungsstrahlengang und mit einem zweiten Beleuchtungsstrahlengang Beleuchtungslicht für den Objektbereich bereitstellen kann, wobei zum Bereitstellen von Licht in dem ersten Beleuchtungsstrahlengang eine erste Lichtaustrittseinheit vorgesehen ist und in dem zweiten Beleuchtungsstrahlengang eine Leuchtfeldblende angeordnet ist, und wobei der erste Beleuchtungsstrahlengang eine Beleuchtungsoptik aufweist, welche die Lichtaustritts-Ebene der ersten Lichtaustrittseinheit oder eine zu dieser Lichtaustritts-Ebene konjugierte Ebene in eine erste Bildebene abbildet.

Ein Operationsmikroskop der eingangs genannten Art ist aus der DE 40 28 605 C2 bekannt. Dieses Operationsmikroskop enthält eine Beleuchtungseinrichtung, bei der über das Austrittsende eines Lichtleiters senkrecht zur optischen Achse des Mikroskop-Hauptobjektivs Beleuchtungslicht bereitgestellt wird. Über ein erstes und ein zweites Umlenkelement auf der dem Objektbereich abgewandten Seite des Mikroskop-Hauptobjektivs wird dieses Beleuchtungslicht mit einem Strahlengang, der parallel zur optischen Achse des Mikroskop-Hauptobjektivs ist, zum Mikroskop-Hauptobjektiv hin umgelenkt. Das Beleuchtungslicht wird so mit einem ersten Strahlengang durch das Mikroskop-Hauptobjektiv zum Objektbereich geführt, der zu den optischen Achsen der Beobachtungsstrahlengänge des Mikroskops achsnah verläuft, und mit einem zweiten Strahlengang, der den Objektbereich mit Schräglicht beleuchtet und einen achsfernen Verlauf im Bezug auf die optischen Achsen der Beobachtungsstrahlengänge des Operationsmikroskops hat. Das Leuchtfeld entspricht dabei dem mittels der Beleuchtungsoptik erzeugten Bild einer am Austrittsende des Lichtleiters angeordneten Leuchtfeldblende im Objektbereich.

Beim Einsatz von Operationsmikroskopen in der Chirurgie werden je nach medizinischer Fachrichtung verschiedene Forderungen an die Beleuchtung des Operationsfeldes gestellt: Bei Operationen in der Hals-, Nasen-, Ohren- und Neurochirurgie wird das Operationsfeld im Bezug auf die Beobachtungsstrahlengänge mit achsnaher Schrägbeleuchtung ausgeleuchtet, damit insbesondere enge, tiefe Körperhöhlen ohne Schattenbildung ausgeleuchtet werden können. Bei mikrochirurgischen Eingriffen am Auge wird diffus von der Netzhaut reflektiertes Beleuchtungslicht verwendet, das einem Operateur, der die Linse eines Patientenauges untersucht, als rötliches Durchlicht erscheint, um transparente Strukturen im vorderen Bereich eines Patientenauges sichtbar zu machen.

Bei ophthalmologischen Operationen ist es günstig, wenn ein Operateur in einem Operationsmikroskop die Möglichkeit hat, in Bezug auf die optische Achse der Beobachtungsstrahlengänge das Operationsfeld mit Beleuchtungslicht unter unterschiedlichen Winkeln auszuleuchten, um auf diese Weise den Kontrast des Beobachtungsbildes einstellen zu können.

In der DE 103 47 732 A1 ist eine Beleuchtungseinrichtung für ein Ophthalmo-Operationsmikroskop beschrieben, die einen Beleuchtungsstrahlengang aufweist, der auf der Retina eines untersuchten Patientenauges eine Abbildung der Beleuchtungspupille der Beleuchtungseinrichtung bewirkt.

Die DE 10 2004 050 651 A1 offenbart ein Operationsmikroskop mit einer Beleuchtungseinrichtung, bei dem in den stereoskopischen Beobachtungsstrahlengängen ein Strahlteilerelement vorgesehen ist, um den Beobachtungsstrahlengängen koaxial Beleuchtungslicht zu überlagern, das durch das Mikroskop-Hauptobjektiv zum Objektbereich gelenkt wird.

In der nicht veröffentlichten deutschen Patentanmeldung Nr. 10 2006 013 761 ist ein Operationsmikroskop mit einer Beleuchtungseinrichtung beschrieben, die Beleuchtungslicht mit einem Beleuchtungsstrahlengang bereitstellt, der durch das Mikroskop-Hauptobjektiv zum Objektbereich geführt ist. Die Beleuchtungseinrichtung umfasst eine köhlersche Beleuchtungsoptik. In dem Beleuchtungsstrahlengang ist eine Reflexblende angeordnet. Die Reflexblende bewirkt eine Abschattung von solchem Beleuchtungslicht, das aufgrund von Reflexionen am Mikroskop-Hauptobjektiv in die Beobachtungsstrahlengänge des Operationsmikroskops gelangen würde. Auf diese Weise können störende Reflexe im Beobachtungsbild des Operationsmikroskops vermieden werden.

Aus der DE 33 39 172 C2 ist ein Ophthalmo-Operationsmikroskop mit einer Beleuchtungseinrichtung bekannt, die eine in den Beleuchtungsstrahlengang ein- und aussschwenkbare Retinaschutzblende umfasst. Die Retinaschutzblende ist als ringförmige Blende ausgebildet. Im eingeschwenkten Zustand befindet sie sich in einer zur Objektebene des Operationsmikroskops konjugierten Ebene und blockiert Beleuchtungslicht bzw. schwächt solches Beleuchtungslicht ab, das sonst durch die Iris eines Patientenauges auf dessen Retina gelangen kann.

In der DE 43 44 770 A1 ist ein Operationsmikroskop beschrieben, das eine Beleuchtungseinrichtung enthält, die es ermöglicht, den Objektbereich in unterschiedlichen Konfigurationen für Beleuchtungslicht auszuleuchten. Die Beleuchtungseinrichtung kann zum einen Beleuchtungslicht über eine Leuchtfeldblende bereitstellen, die durch das Mikroskop-Hauptobjektiv in den Objektbereich abgebildet wird. Zum anderen kann die Beleuchtungseinrichtung den Objektbereich mit Beleuchtungslicht in einer Konfiguration beleuchten, bei dem die Glühwendel einer Lampe oder das Austrittsende eines Lichtleiters durch das Mikroskop-Hauptobjektiv in den Objektbereich abgebildet wird.

Die DE 20 2004 019 849 U1 offenbart ein Operationsmikroskop, das die Beleuchtung eines Objektbereichs mit unterschiedlich konfiguriertem Beleuchtungslicht durch das Mikroskop-Hauptobjektiv hindurch ermöglicht. Bei einer ersten Konfiguration für Beleuchtungslicht wird entsprechend der DE 43 44 770 A1 über das Mikroskop-Hauptobjektiv eine Leuchtfeldblende in den Objektbereich abgebildet. Das Beleuchtungslicht entsprechend der zweiten Konfiguration durchsetzt einen Diffusor, der bei einer Lichtquelle angeordnet ist, welche in den Objektbereich abgebildet wird.

Die DE 196 11 044 A1 offenbart ein Operationsmikroskop mit einer Lichtquelle; einem ersten optischen Beleuchtungssystem zum Projizieren von Licht von der Lichtquelle auf ein zu untersuchendes Auge aus einer ersten Richtung heraus und einem zweiten optischen Beleuchtungssystem zum Projizieren von Licht von der Lichtquelle auf das zu untersuchende Auge aus einer Richtung heraus, die von der ersten Richtung verschieden ist; sowie einem optischen Beobachtungssystem, um dadurch das zu untersuchende Auge zu betrachten, das von wenigstens entweder von dem ersten optischen Beleuchtungssystem und dem zweiten optischen Beleuchtungssystem beleuchtet wird.

Die EP 1 109 046 A1 offenbart eine Beleuchtungseinrichtung für ein Operationsmikroskop, mit der die Beleuchtung durch das Mikroskopobjektiv erfolgt. Bei der Beleuchtungseinrichtung können mit Hilfe von zwei unabhängig voneinander verschiebbaren Reflexionselementen die Winkel des einfallenden Lichtes mit der optischen Achse des Mikroskopobjektivs und auch die Intensität der verschiedenen Lichtstrahlen unabhängig voneinander verändert werden.

Aufgabe der Erfindung ist es, ein Operationsmikroskop bereitzustellen, das sich zum Einsatz in der Ophthalmologie eignet und ermöglicht, transparente Strukturen im vorderen Abschnitt eines Patientenauges mit gutem Kontrast sichtbar zu machen.

Diese Aufgabe wird durch ein Operationsmikroskop der eingangs genannten Art gelöst, bei dem in dem zweiten Beleuchtungsstrahlengang eine Beleuchtungsoptik vorgesehen ist, welche die Leuchtfeldblende in eine, von der ersten Bildebene verschiedene zweite Bildebene abbildet.

Der erste Beleuchtungsstrahlengang ist relativ zu wenigstens einer optischen Achse des Beobachtungsstrahlenganges des Operationsmikroskops mit einem achsnahen Verlauf zum Objektbereich gerührt. Auf diese Weise lässt sich an einem Patientenauge ein roter Reflex mit guter Homogenität erzeugen.

Der zweite Beleuchtungsstrahlengang ist relativ zu wenigstens einer optischen Achse des Operationsmikroskops mit einem achsfernen Verlauf zum Objektbereich geführt. Auf diese Weise wird eine gute Plastizität eines Bildes des Objektbereichs im Operationsmikroskop bewirkt, da das schräg einfallende Beleuchtungslicht an Strukturen im Objektbereich Schattenbildung hervorruft.

Die zweite Bildebene, in der das Bild der Leuchtfeldblende im zweiten Beleuchtungsstrahlengang liegt, fällt mit der Objektebene des Operationsmikroskops zusammen oder liegt in deren Nähe. Auf diese Weise wird eine saubere Begrenzung des Leuchtfeldes im Objektbereich bewirkt.

Insgesamt wird auf diese Weise ein Operationsmikroskop geschaffen, bei dem ein großes Leuchtfeld mit sauberer Begrenzung gewährleistet ist und bei dem gleichzeitig ein lichtstarker roter Reflex an einem untersuchten Patientenauge erzeugt werden kann.

In Weiterbildung der Erfindung durchsetzt bei dem Operationsmikroskop der erste Beleuchtungsstrahlengang und/oder der zweite Beleuchtungsstrahlengang das Mikroskop-Hauptobjektiv. Auf diese Weise wird ein großer freier Arbeitsbereich zwischen Mikroskop-Hauptobjektiv und Objektbereich ermöglicht.

In Weiterbildung der Erfindung ist in dem Operationsmikroskop ein erstes oder ein zweites Umlenkelement vorgesehen, das achsnahes bzw. achsfernes Beleuchtungslicht durch das Mikroskop-Hauptobjektiv zum Objektbereich lenkt. Auf diese Weise wird ein im Bezug auf die optische Achse des Mikroskop-Hauptobjektivs seitliches Einkoppeln von Beleuchtungslicht in das System ermöglicht.

In Weiterbildung der Erfindung ist das erste Umlenkelement als teildurchlässiger Spiegel ausgebildet, der von einem Beobachtungsstrahlengang des Operationsmikroskops durchsetzt wird. Der teildurchlässige Spiegel kann insbesondere als geometrischer oder physikalischer Strahlteiler ausgeführt sein. Auf diese Weise kann ein Strahlengang für Beleuchtungslicht ohne Vignettierung nahe bei den Beobachtungsstrahlengängen des Operationsmikroskops geführt werden.

In Weiterbildung der Erfindung dient das erste Umlenkelement dazu, einem stereoskopischen Beobachtungsstrahlengang, vorzugsweise einem linken und einem rechten stereoskopischen Beleuchtungsstrahlengang, achsnahes Beleuchtungslicht koaxial zu überlagern. Auf diese Weise wird Beleuchtungslicht koaxial zum Beobachtungsstrahlengang auf den Objektbereich gerührt. So kann in der Ophthalmologie an einem Patientenauge ein besonders lichtstarker und homogener, roter Reflex erzielt werden.

In Weiterbildung der Erfindung ist ein zweites Umlenkelement vorgesehen, das Beleuchtungslicht durch das Mikroskop-Hauptobjektiv zum Objektbereich lenkt. Auf diese Weise wird eine kompakte Bauform des Operationsmikroskops ermöglicht.

In Weiterbildung der Erfindung ist die zweite Leuchtfeldblende als Blende mit variierbarer, insbesondere einstellbarer Blendenöffnung ausgebildet. Auf diese Weise kann die Größe des Leuchtfeldes im Objektbereich eingestellt werden.

In Weiterbildung der Erfindung ist bei der Leuchtfeldblende eine weitere Blende angeordnet. Vorzugsweise ist die weitere Blende als Retinaschutzblende ausgebildet. Auf diese Weise kann die Belastung der Retina mit Beleuchtungslicht bei ophthalmologischen Operationen reduziert bzw. unterbunden werden.

In Weiterbildung der Erfindung ist in dem zweiten Beleuchtungsstrahlengang eine Aperturblende angeordnet. Vorzugsweise ist diese Aperturblende einstellbar ausgeführt.

Mit dieser Maßnahme kann die Helligkeit des Leuchtfeldes bei dem Operationsmikroskop variiert werden, ohne dass es hierfür der Steuerung einer Lichtquellenleistung bedarf.

In Weiterbildung der Erfindung ist in dem ersten Beleuchtungsstrahlengang eine Blende mit variierbarer, insbesondere einstellbarer Blendenöffnung ausgebildet, um die Lichtmenge in dem Strahlengang zu steuern. Auf diese Weise kann die Helligkeit des Rotreflexes in der Ophthalmologie ohne Steuerung einer Lichtquellenleistung verändert werden.

In Weiterbildung der Erfindung ist zur Einstellung der Blende in dem zweiten Beleuchtungsstrahlengang und/oder der Aperturblende und/oder der Blende in dem ersten Beleuchtungsstrahlengang ein gemeinsames benutzerbetätigbares Bedienelement vorgesehen. Auf diese Weise wird ein ergonomisch günstig handhabbares Operationsmikroskop bereitstellt.

In Weiterbildung der Erfindung ist das benutzerbetätigbare Bedienelement als Drehknopf ausgebildet. Auf diese Weise wird eine sichere Einstellung von Blendenkonfigurationen ermöglicht, wobei Fehlbedienungen weitestgehend ausgeschlossen werden können, die eine Schädigung eines Patientenauges mit Beleuchtungslicht zur Folge hätten.

In Weiterbildung der Erfindung ist der Drehknopf mit einer Welleneinheit gekoppelt, die über eine erste Steuerkurve sowie einer ersten Abtriebseinheit mit der Blende im ersten Beleuchtungsstrahlengang in Wirkverbindung steht, die über eine zweite Steuerkurve sowie eine zweite Abtriebseinheit mit der verstellbaren Blende im zweiten Beleuchtungsstrahlengang in Wirkverbindung steht, und die über eine dritte Steuerkurve sowie eine dritte Abtriebseinheit mit der Aperturblende in Wirkverbindung steht. Auf diese Weise wird eine besonders kleine Bauform der Beleuchtungseinrichtung ermöglicht und es wird ohne elektrische Antriebe eine zuverlässige Funktionsweise des Operationsmikroskops gewährleistet.

In Weiterbildung der Erfindung ist dem ersten Beleuchtungsstrahlengang und dem zweiten Beleuchtungsstrahlengang eine gemeinsame Lichtquelle zugeordnet. Auf diese Weise wird eine kostengünstige Bauform für ein Operationsmikroskop mit Beleuchtungseinrichtung geschaffen.

In Weiterbildung der Erfindung ist bei dem Operationsmikroskop ein Lichtleiter mit Verzweigung vorgesehen, der das Licht der gemeinsamen Lichtquelle dem ersten Beleuchtungsstrahlengang und dem zweiten Beleuchtungsstrahlengang zuführt. Auf diese Weise kann bei dem Operationsmikroskop die Lichtquelle mit Abstand von der Optik des Operationsmikroskops angeordnet werden.

In Weiterbildung der Erfindung weist der Lichtleiter eine erste Lichtaustrittseinheit und eine zweite Lichtaustrittseinheit auf. Auf diese Weise ist es möglich, unter Verwendung einer einzigen Lichtquelle Beleuchtungslicht für einen achsnahen und einen achsfernen Beleuchtungsstrahlengang gleichzeitig bereitzustellen.

In Weiterbildung der Erfindung ist an der ersten Lichtaustrittseinheit eine erste Beleuchtungspupille und eine zweite Beleuchtungspupille ausgebildet. Auf diese Weise lässt sich eine optimierte Rotreflexbeleuchtung für beide stereoskopische Beobachtungsstrahlengänge eines Operationsmikroskop erzielen.

In Weiterbildung der Erfindung ist an der zweiten Lichtaustrittseinheit eine einzige Beleuchtungspupille ausgebildet. Auf diese Weise ist im Objektbereich des Operationsmikroskops ein homogenes Leuchtfeld darstellbar.

In Weiterbildung der Erfindung ist in dem ersten Beleuchtungsstrahlengang des Operationsmikroskops eine Reflexblende vorgesehen, die Beleuchtungsstrahlen abschattet, welche durch Reflexionen an optischen Elementen im Operationsmikroskop, insbesondere Reflexionen am Mikroskop-Hauptobjektiv, störendes Streulicht in den Beobachtungsstrahlengängen des Operationsmikroskops hervorrufen. Auf diese Weise kann der Kontrast des Bildes, das für eine Beobachtungsperson im Binokulartubus des Operationsmikroskops sichtbar ist, maximiert werden.

In Weiterbildung der Erfindung ist die Reflexblende im ersten Beleuchtungsstrahlengang des Operationsmikroskops derart angeordnet, dass keine Beobachtungsstrahlen im Operationsmikroskop ausgeblendet werden und kein Beschnitt des Bildes der Leuchtfeldblende in der zweiten Bildebene durch die Reflexblende erfolgt. Auf diese Weise wird ein lichtstarkes Beobachtungsbild im Operationsmikroskop bewirkt, in dem keine störenden Reflexe auftreten, wobei das Leuchtfeld in allen Bereichen gleichmäßig ausgeleuchtet wird.

In Weiterbildung der Erfindung ist die Beleuchtungseinrichtung in dem Operationsmikroskop derart ausgelegt, dass auf der Retina eines idealen Patientenauges mit Beleuchtungslicht aus dem ersten Beleuchtungsstrahlengang wenigstens ein Beleuchtungsspot, vorzugsweise ein erster und ein zweiter Beleuchtungsspot mit einem Durchmesser im Bereich 0,5 mm bis 1,5 mm erzeugt werden kann. Auf diese Weise lässt sich an einem Patientenauge ein roter Reflex erzeugen, der mit exzellentem Kontrast im Operationsmikroskop sichtbar ist.

Vorteilhafte Ausführungsformen der Erfindung sind in den Zeichnungen dargestellt und werden nachfolgend beschrieben.

Es zeigen:
- Fig. 1: einen schematischen Schnitt eines ersten Operationsmikroskops mit einer Beleuchtungseinrichtung, die einen ersten Beleuchtungsstrahlengang mit achsnahem Beleuchtungslicht für Rotreflexbeleuchtung und einen zweiten Beleuchtungsstrahlengang mit achsfernem Beleuchtungslicht für Umfeldbeleuchtung bereitstellt, der jeweils durch das Mikroskop-Hauptobjektiv des Operationsmikroskop geführt ist;
- Fig. 2: einen schematischen Schnitt entlang der Linie II - II des Operationsmikroskops aus Fig. 1 mit einem Beleuchtungsstrahlengang für Umfeldbeleuchtung;
- Fig. 3: einen schematischen Schnitt entlang der Linie III - III des Operationsmikroskops aus Fig. 1 mit dem ersten Beleuchtungsstrahlengang für Rotreflexbeleuchtung;
- Fig.4: eine Reflexblende in dem ersten Beleuchtungsstrahlengang für Rotreflexbeleuchtung;
- Fig. 5: eine 3-dimensionale Ansicht einer Baugruppe der Beleuchtungseinrichtung mit einem Drehknopf;
- Fig. 6: verschiedene Einstellungen des Drehknopfs der Baugruppe;
- Fig.7: einen Lichtleiter mit Lichtquelle, um die Beleuchtungseinrichtung des Operationsmikroskops mit Licht zu versorgen;
- Fig. 8: ein erstes Austrittsende des Lichtleiters für Licht mit zwei Beleuchtungspupillen;
- Fig.9: ein zweites Austrittsende des Lichtleiters für Licht mit einer einzigen Beleuchtungspupille;
- Fig. 10: einen schematischen Schnitt eines zweiten Operationsmikroskops mit einer Beleuchtungseinrichtung, die einen ersten Beleuchtungsstrahlengang mit achsnahem Beleuchtungslicht bereitstellt, der am Mikroskop-Hauptobjektiv vorbei zum Objektbereich geführt ist, und eine zweiten Beleuchtungsstrahlengang umfasst, der das Mikroskop-Hauptobjektiv durchsetzt;
- Fig. 11: einen schematischen Schnitt eines dritten Operationsmikroskops mit einer Beleuchtungseinrichtung, bei der ein erster Beleuchtungsstrahlengang mit achsfernen Beleuchtungslicht am Mikroskop-Hauptobjektiv vorbei zum Objektbereich geführt ist; und
- Fig. 12: einen schematischen Schnitt eines vierten Operationsmikroskop mit einer Beleuchtungseinrichtung, bei der ein erster Beleuchtungsstrahlengang mit achsnahem Beleuchtungslicht durch das Mikroskop-Hauptobjektiv zum Objektbereich geführt ist und ein zweiter Beleuchtungsstrahlengang achsfernes Beleuchtungslicht an Mikroskop-Hauptobjektiv vorbei für den Objektbereich bereitstellt.

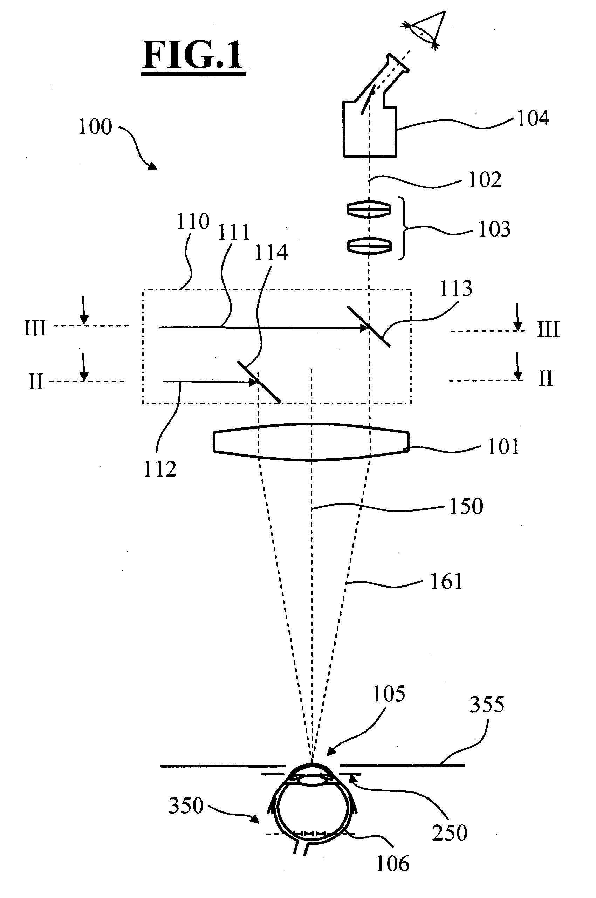

Das Operationsmikroskop 100 in Fig. 1 hat ein Mikroskop-Hauptobjektiv 101 mit einer optischen Achse 150, welches von einem stereoskopischen Beobachtungsstrahlengang 102 durchsetzt wird. In der Fig. 1 ist der linke Teilstrahlengang des stereoskopischen Beobachtungsstrahlengangs 102 gezeigt. Dieser Teilstrahlengang hat eine optische Achse 161. Das Operationsmikroskop 100 ermöglicht einer Beobachtungsperson über ein Vergrößerungssystem 103 und einen Binokulartubus 104 einen Objektbereich 105 in einer Objektebene 355 zu untersuchen. Das Operationsmikroskop 100 ist als Ophthalmo-Operationsmikroskop ausgelegt und eignet sich insbesondere zur Untersuchung eines Patientenauges 106.

Das Operationsmikroskop 100 enthält eine Beleuchtungseinrichtung 110, die in Bezug auf den stereoskopischen Beobachtungsstrahlengang 102 einen Beleuchtungsstrahlengang 111 für achsnahe Beleuchtung und einen Beleuchtungsstrahlengang 112 für achsferne Beleuchtung des Objektbereichs bereitstellt.

Die Beleuchtungseinrichtung 110 umfasst einen für Licht teildurchlässigen Spiegel 113, der als erstes Umlenkelement wirkt, um dem stereoskopischen Beobachtungsstrahlengang zu dessen optischen Achsen 102 Beleuchtungslicht achsnah und koaxial zu überlagern. Die Beleuchtungseinrichtung 110 enthält einen Spiegel 114 als zweites Umlenkelement, der Beleuchtungslicht durch das Mikroskop-Hauptobjektiv 101 mit achsfernem Verlauf, d.h. unter einem Winkel zur optischen Achse der Beobachtungsstrahlengänge, zum Objektbereich 105 lenkt.

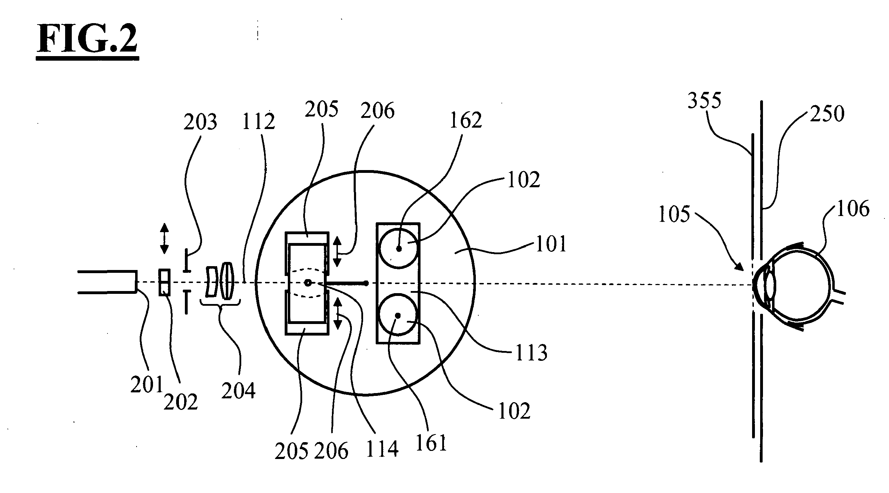

Die Fig. 2 zeigt einen Schnitt entlang der II - II aus Fig. 1. Soweit in Fig. 2 die gleichen Baugruppen bzw. Gegenstände wie in Fig. 1 zu erkennen sind, werden die gleichen Bezugszeichen wie in Fig. 1 verwendet.

Der stereoskopische Beobachtungsstrahlengang 102 durchsetzt das Mikroskop-Hauptobjektiv 101 mit einem linken und einem rechten Teilstrahlengang mit optischen Achsen 161 und 162. Das Mikroskop-Hauptobjektiv 101 hat eine Brennweite von f=200 mm. Eine günstige Brennweite für das Mikroskop-Hauptobjektiv ist aber auch die Brennweite f=175 mm oder f=225 mm.

Um achsfernes Beleuchtungslicht zum Objektbereich zu führen, wird das Licht aus einer Lichtaustrittseinheit in Form eines Lichtleiter-Austrittsendes 201 über eine einstellbare Blende 202 und eine Leuchtfeldblende 203 durch eine Beleuchtungsoptik 204 dem Spiegel 114 zugeführt, der es durch das Mikroskop-Hauptobjektiv 101 zum Objektbereich 105 lenkt.

Die Blende 202 kann für eine Wirkung als Retinaschutzblende eingestellt werden. In dieser Einstellung befindet sich eine Ringblende in dem Beleuchtungsstrahlengang 112, die wie in der Patentschrift DE 33 39 172 C2 angegeben sein kann.

Die Leuchtfeldblende 203 wird in eine Bildebene 250 abgebildet. Diese Bildebene 250 liegt nahe bei, jedoch nicht genau, in der Objektebene 355 des Operationsmikroskops 100 aus Fig. 1. Auf diese Weise ergibt sich eine Begrenzung des Leuchtfeldes in der Objektebene, das in seinem Randbereich nicht abrupt sondern weich von Hell nach Dunkel übergeht. Damit ist für eine Beobachtungsperson im Okulareinblick auch im Randbereich des Leuchtfeldes ein angenehmer Seheindruck gewährleistet. Es sei bemerkt, dass grundsätzlich auch eine scharfe Abbildung der Leuchtfeldblende in die Objektebene eines Operationsmikroskops vorgesehen werden kann. Diese Maßnahme ist jedoch auch mit dem Nachteil verbunden, dass die Lichtdurchtrittsöffnung der Leuchtfeldblende, die häufig fertigungsbedingt mechanische Defekte hat, als vergrößertes Bild in die Objektebene des Operationsmikroskops abgebildet wird, wo diese Defekte dann sichtbar sind.

Ist die Blende 202 für die Wirkung als Retinaschutzblende eingestellt, wird vermieden, dass Beleuchtungslicht in das Innere des Patientenauges 106 zum Augenhintergrund gelangt, wo es unter Umständen schädigende Wirkung entfalten könnte. Dabei wird die Blende 202 in den Objektbereich 105 abgebildet, d.h. bei der in Fig. 1 gezeigten Einstellung des Operationsmikroskops 100 also in etwa auf die Hornhaut des Patientenauges 106. Zwischen dem Mikroskop-Hauptobjektiv 101 und dem Spiegel 114 ist eine einstellbare Blende 205 vorgesehen, die entsprechend dem Doppelpfeil 206 verstellt werden kann, um den Beleuchtungsstrahlengang aus dem Lichtleiter-Austrittsende 201 freizugeben oder zu versperren. Die Blende 205 kann also so verstellt werden, dass sie den Beleuchtungsstrahlengang kontinuierlich oder in Stufen freigeben bzw. versperren kann.

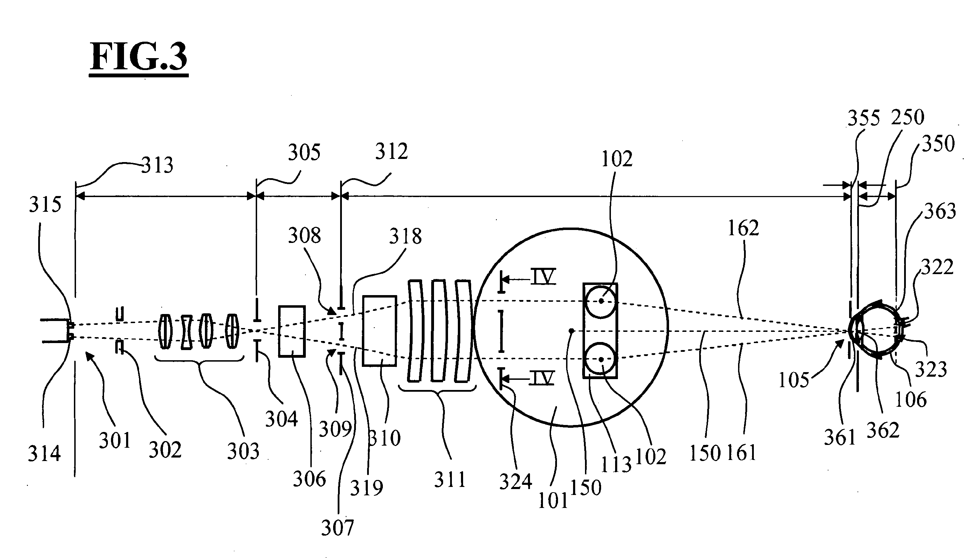

Die Fig. 3 zeigt einen schematischen Schnitt des Operationsmikroskops entlang der Linie III - III aus Fig. 1. Soweit die in Fig. 3 gezeigten Baugruppen des Operationsmikroskops Baugruppen, aus Fig. 1 entsprechen, sind zu deren Bezeichnung die gleichen Bezugszeichen verwendet.

Um achsnahes Beleuchtungslicht zum Objektbereich 105 zu führen, wird mit dem Licht aus einer Lichtaustrittseinheit in Form eines Lichtleiter-Austrittsendes 301 über eine einstellbare bzw. schließbare Aperturblende 302 und eine erste Beleuchtungsoptik 303 eine Leuchtfeldblende 304 ausgeleuchtet. Diese Leuchtfeldblende 304 ist in einer Ebene 305 angeordnet. Von der Leuchtfeldblende 304 wird das Beleuchtungslicht mit einem gefalteten Beleuchtungsstrahlengang über ein Spiegelelement 306, eine Doppelblende 307 mit zwei Blendenöffnungen 308 und 309 zu einem Spiegelelement 310 gelenkt. Dieses Spiegelelement 310 lenkt das Beleuchtungslicht über eine zweite Beleuchtungsoptik 311 senkrecht zur optischen Achse 150 des Mikroskop-Hauptobjektivs 101 durch eine als Streulichtblende wirkende Reflexblende 324 zu dem Umlenkelement 113, das als Strahlteiler ausgebildet ist und vom dem linken und dem rechten stereoskopischen Beobachtungsstrahlengang 102 des Operationsmikroskops durchsetzt wird. Das Umlenkelement 113 lenkt das Beleuchtungslicht parallel zur optischen Achse 150 des Mikroskop-Hauptobjektivs 101 durch das Mikroskop-Hauptobjektiv 101 koaxial mit dem stereoskopischen Beobachtungsstrahlengang 102 zum Objektbereich 105.

Die Doppelblende 307 wirkt als Aperturblende. Sie ist in einer Ebene 312 angeordnet, welche zur Lichtaustrittsebene 313 des Lichtleiter-Austrittsendes 301 konjugiert ist. An dem Lichtleiter-Austrittsende 301 sind eine erste Lichtaustrittseinheit 314 mit einer ersten Beleuchtungspupille und eine zweite Lichtaustrittseinheit 315 mit einer zweiten Beleuchtungspupille ausgebildet. Diese erste und zweite Lichtaustrittseinheit 314, 315 wird mit der ersten Beleuchtungsoptik 303 vergrößert in die Ebene 312 der Doppelblende 307 mit den Blendenöffnungen 308 und 309 abgebildet.

Die Leuchtfeldblende 304 wird über die Spiegelelemente 306, 310 mit der zweiten Beleuchtungsoptik 311 durch das Mikroskop-Hauptobjektiv 101 in die Bildebene 250 im Objektbereich 105 abgebildet. D.h., die Ebene 305 der Leuchtfeldblende 304 ist zu dieser Bildebene 250 konjugiert. Damit ergibt sich wiederum eine saubere Begrenzung des mit diesem Beleuchtungsstrahlengang hervorgerufenen Leuchtfeldes im Objektbereich.

Die Blendenöffnungen 308, 309 der Doppelblende 307 werden über das Spiegelelement 310, die zweite Beleuchtungsoptik 311 durch das Mikroskops-Hauptobjektiv 101 und das Patientenauge 106 in eine von der Bildebene 250 der Leuchtfeldblende 304 verschiedene Bildebene 350 abgebildet. Die Bildebene 350 ist damit zu der Ebene 312 der Doppelblende 307 und zu der Lichtaustrittsebene 313 des Lichtleiter-Austrittsendes konjugiert. Die optische Achse 318, 319 des Abbildungsstrahlenganges für die Blendenöffnungen 308, 309 der Doppelblende 307 wird mit dem Umlenkelement 113 in die optischen Achsen 161, 162 des linken und rechten stereoskopischen Beobachtungsstrahlenganges 102 überführt.

Zwischen Mikroskop-Hauptobjektiv 101 und dem Patientenauge 106 ist dieser Abbildungsstrahlengang parallel oder in etwa parallel. D.h., ohne die brechende Wirkung von Cornea 361 und Linse 362 des Patientenauges 106 liegt die Bildebene 350 für die Doppelblende 307 im unendlichen, bzw. sie ist von der Brennebene, d.h. der Objektebene 355 des Mikroskop-Hauptobjektivs 101 weit beabstandet. Es ist möglich, dass der entsprechende Abbildungsstrahlengang für die Doppelblende 307 austrittsseitig des Mikroskop-Hauptobjektivs 101 leicht aufgeweitet verläuft. Dann ist die Bildebene 350 ohne die brechende Wirkung von Cornea 361 und Linse 362 des Patientenauges 106 auf der dem Objekt abgewandten Seite des Mikroskop-Hauptobjektives 101 angeordnet und ist virtueller Natur. Verläuft der Abbildungsstrahlengang austrittsseitig des Mikroskop-Hauptobjektivs 101 leicht konvergent, ist die Bildebene 350 reell und befindet sich auf der dem Objekt zugewandten Seite des Mikroskop-Hauptobjektivs 101.

Wird mit dem Operationsmikroskop ein Patientenauge 106 untersucht, so bewirken Cornea 361 und natürliche Linse 362 im Auge, dass der parallele oder in etwa parallele Beleuchtungsstrahlengang, der entlang der optische Achsen 161, 162 verläuft, gebündelt wird. Bei einem auf unendlich adaptierten, rechtsichtigen Patientenauge 106 haben die Brechkraft von Cornea 361 und natürlichen Linse 362 zur Folge, dass der Fokus des Beleuchtungsstrahlengagens auf der Retina 363 des Patientenauges 106 liegt, also die Bildebene 350 bei der Retina 363 des Patientenauges 106 angeordnet ist. Dann befindet sich auf der Retina 363 des Patientenauges 106 ein Bild der Doppelblende 307. Auf der Retina 363 des Patientenauges 106 entsteht so ein erster Beleuchtungsspot 322 und ein zweiter Beleuchtungsspot 323.

Die Bemaßung der Größe der Lichtaustrittseineiten 314, 315 und deren gegenseitiger Abstand, die Bemaßung der Blendenöffnungen 308, 309 und deren gegenseitiger Abstand in der Doppelblende 307 sowie die Dimensionierung der Abbildungsoptik 311 im Beleuchtungsstrahlengang und die Brennweite des Mikroskop-Hauptobjektivs 101 ist bei dem Operationsmikroskop so gewählt, dass bei Untersuchen eines idealen Patientenauges, das in seinen optischen Eigenschaften demjenigen des schematischen Auges von Gullstrand entspricht, wie es auf S. 83 des "ABC der Optik, Edition Leipzig, Verlag Werner Dausien, Hanau/Main, 1961" gegeben ist, auf der Retina auf den optischen Achsen 161, 162 der Beobachtungsstrahlengänge zwei Beleuchtungsspots 322, 323 entstehen, deren Durchmesser im Bereich 0.5mm bis 1.5mm liegt. Dabei erweisen sich kleine Beleuchtungsspots als besonders günstig, da sie Voraussetzung für ein besonders kontrastreiches Rotreflexbild des Vordergrundes eines Patientenauges sind, das sich für eine Beobachtungsperson im Okulareinblick des Operationsmikroskops bietet. Die Größe eines Beleuchtungsspots auf der Retina darf aber auch nicht zu groß sein:
Mit zunehmender Größe des Beleuchtungsspots auf dem Augenhintergrund nimmt der Kontrast des Rotreflexbildes vom Vordergrund des Patientenauges im Operationsmikroskop ab.

Besonders kleine Beleuchtungsspots werden erzielt, indem die Doppelblende 307 bzw. die Lichtaustrittsebene 313 des Lichtleiter-Austrittsendes 301 auf die Retina 363 des Patientenauges 106 abgebildet wird, d.h., wenn die Bildebene 350 auf der Retina 363, also dem Hintergrund des Patientenauges 106 liegt, das sich für eine Beobachtungsperson im Okulareinblick des Operationsmikroskops bietet.

Eine zu kleine Spotgröße ist allerdings nachteilhaft: Ist die Spotgröße zu klein, so verschlechtert sich die Homogenität des roten Reflexes d.h. der rote Reflex wird in seinem peripheren Bereich nach außen dunkler. Außerdem birgt ein zu kleiner Beleuchtungsspot auf der Retina wegen der dort herrschenden hohen Bestrahlungsstärke des Beleuchtungslichts die Gefahr, dass die Netzhaut des Patientenauges geschädigt wird.

Es versteht sich, dass der Beleuchtungsstrahlengang für achsnahes Beleuchtungslicht nicht unbedingt gefaltet ausgeführt sein muss, wenn ein entsprechend größeres Bauvolumen in Kauf genommen wird. Weiter ist zu bemerken, dass die Spiegelelemente 306 und 310 bzw. die Beleuchtungsoptik 303, 311 auch mehrteilig ausgeführt werden können, so dass das in den linken und rechten stereoskopischen Beobachtungsstrahlengang eingekoppelte Beleuchtungslicht über unterschiedliche Spiegel bzw. Objektive geführt wird.

Darüber hinaus sei bemerkt, dass der Lichtleiter-Austrittsende 301 in Ebene 312 der Doppelblende 307 angeordnet werden könnte. Mit dieser Maßnahme wird in Kauf genommen, dass der aus dem Lichtleiter-Austrittsende auftretende Beleuchtungsstrahlengang im Objektbereich kein scharf begrenztes Leuchtfeld bewirkt. Das Beleuchtungssystem kann jedoch auf diese Weise besonders platzsparend gebaut werden.

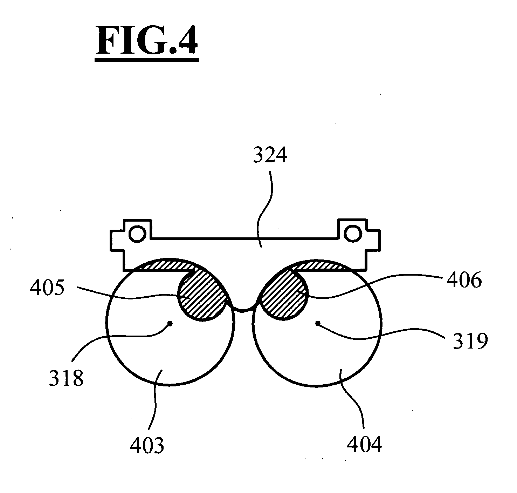

Fig. 4 zeigt einen Schnitt der Reflexblende 324 entlang der Linie IV - IV aus Fig. 3. Der Beleuchtungsstrahlengang bei der Reflexblende 324 hat eine optische Achse 318 und eine optische Achse 319 mit Querschnittsflächen 403 und 404. Aus dem Beleuchtungsstrahlengang werden in den Bereichen 405 und 406 mittels der Reflexblende 324 jene Beleuchtungsstrahlen abgeschattet, welche nach Umlenken durch das Umlenkelement 113 durch Reflexionen am Mikroskop-Hauptobjektiv 101 in Fig. 1 Streulicht hervorrufen, das über das Vergrößerungssystem 103 im Operationsmikroskop 100 aus Fig. 1 eingefangen wird, so dass das für eine Beobachtungsperson im Binokulartubus 104 sichtbare Beobachtungsbild beeinträchtigt.

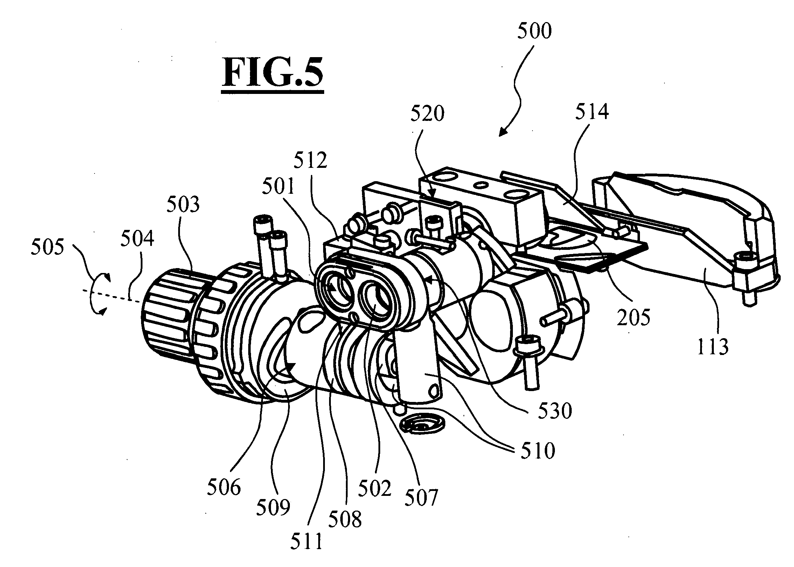

Fig. 5 zeigt eine 3-dimensionale Ansicht einer zentralen Einheit 500 der Beleuchtungseinrichtung 110 aus Fig. 1. Soweit die Baugruppen der Einheit 500 in den Figuren 1, 2, 3 oder 4 zu erkennen sind, werden zu deren Bezeichnung die gleichen Bezugszeichen verwendet. Die Einheit 500 der Beleuchtungseinrichtung hat eine Aufnahme 501 für ein Lichtleiterende, das Licht für achsferne Beleuchtung bereitstellt. Die Einheit 500 umfasst eine Aufnahme 502 für ein Lichtleiterende, aus dem Licht für achsnahes Beleuchtungslicht austritt.

Für das Verstellen der bei Bezugszeichen 520 angeordneten Retinaschutzblende 202 aus Fig. 2 sowie der Blende 205 und der bei Bezugszeichen 530 angeordneten Aperturblende 302 aus Fig. 3 ist bei der Einheit 500 ein Drehknopf 503 vorgesehen, der um eine Achse 504 entsprechend dem Doppelpfeil 505 gedreht werden kann. Der Drehknopf 503 ist mit einer Welle 506 verbunden in denen eine erste Steuerkurve 507, eine zweite Steuerkurve 508 sowie eine dritte Steuerkurve 509 ausgebildet ist. Die erste Steuerkurve 507 ist mit einer ersten Abtriebseinheit 510 in Wirkverbindung, mittels dessen die Blende 302 aus Fig. 3 verstellt werden kann. Die zweite Steuerkurve 508 wirkt auf eine zweite Abtriebseinheit 511, um die Retinaschutzblende 202 aus Fig. 2 zu verstellen. Die dritte Steuerkurve 509 ist mit einem dritten Abtriebselement 512 in Wirkverbindung, welches die Blende 205 aus Fig. 2 steuert.

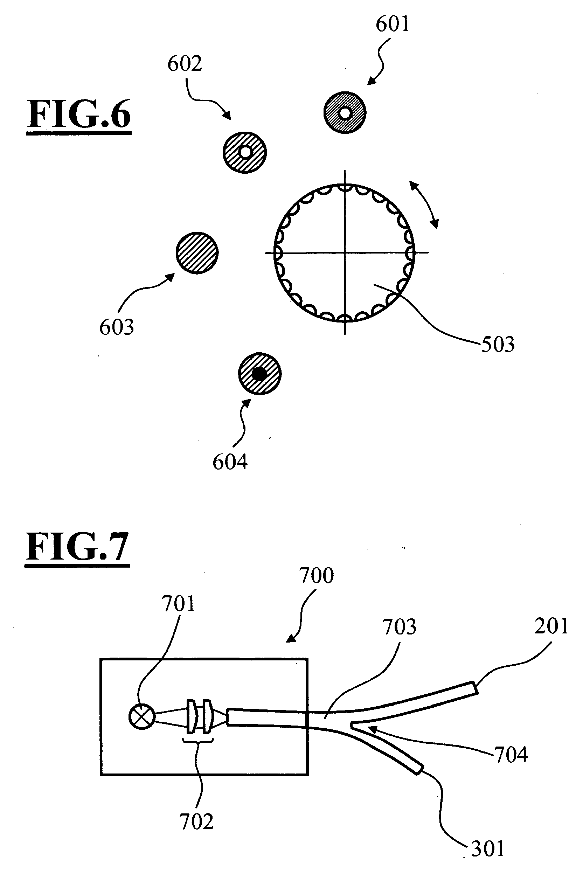

Die Figur 6 zeigt verschiedene mögliche Einstellungen des Drehknopfes 503 aus Fig. 5. In der Einstellung 601 ist der Strahlengang für achsfernes Beleuchtungslicht versperrt und es wird lediglich achsnahes Beleuchtungslicht zum Objektbereich geführt. Beim Verändern der Einstellung des Drehknopfes 503 von der Position 601 in die Position 602 nimmt die Intensität desjenigen Beleuchtungslichtes zu, das bei dem Operationsmikroskop mit achsfernem Verlauf im Bezug auf die optischen Achse der Beobachtungsstrahlenganges zum Objektbereich geführt wird. Dabei wird in der Einstellung 602 sowohl achsnahes als auch achsfernes Beleuchtungslicht bereitgestellt. Weiter kann der Drehknopf 503 in die Position 603 bewegt werden. In dieser Einstellung 603 ist der Strahlengang für achsnahes Beleuchtungslicht unterbrochen und es wird lediglich achsfernes Beleuchtungslicht zum Objektbereich geführt. Schließlich ist für den Drehknopf 503 eine Einstellung 604 möglich. In der Einstellung 604 des Drehknopfes ist der Strahlengang für achsnahes Beleuchtungslicht unterbrochen. In dieser Einstellung wird Beleuchtungslicht entsprechend dem Beleuchtungsstrahlengang für achsfernes Beleuchtungslicht bereitgestellt, wobei die Retinaschutzblende in den Beleuchtungsstrahlengang eingeschaltet ist.

Der Beleuchtungseinrichtung 110 des Operationsmikroskops 100 aus Fig. 1 ist eine Lichtquelle 700 zugeordnet, die in Fig. 7 gezeigt ist. Die Lichtquelle 700 umfasst eine Halogenlampe 701, deren Licht über eine Optikeinheit 702 einem Lichtleiter 703 zugeführt ist. Es ist jedoch insbesondere auch möglich zu Lichterzeugung in der Lichtquelle eine Xenonlampe vorzusehen. Es sei bemerkt, dass als Lichtquelle auch eine auf LEDs basierende Lichtquelle eingesetzt werden kann. Der Lichtleiter 703 hat eine Verzweigung 704, um an einem ersten Austrittsende 201 Beleuchtungslicht für achsferne Beleuchtung bereitzustellen. Der Lichtleiter hat ein zweites Austrittsende 301, aus dem Licht für achsnahe Beleuchtung des Objektbereichs beim Operationsmikroskop 100 aus Fig. 1 austritt.

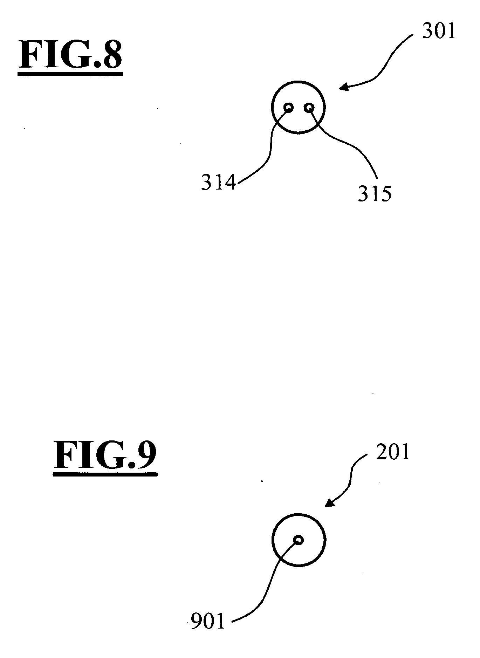

Die Fig. 8 zeigt das Austrittsende 301 des Lichtleiters 703. Austrittsende 301 ist als Lichtaustrittseinheit mit einer ersten Beleuchtungspupille 314 und einer zweiten Beleuchtungspupille 315 ausgebildet.

In Fig. 9 ist das Austrittsende 201 des Lichtleiters 703 gezeigt. Das Austrittsende 201 wirkt ebenfalls als Lichtaustrittseinheit. Hier gibt es jedoch nur eine Beleuchtungspupille 901.

Die Beleuchtungseinrichtung 110 des in Fig. 1 gezeigten Operationsmikroskops 100 ermöglicht eine äußerst vielseitige Beleuchtung des Objektbereichs 105 des Operationsmikroskops 100: Durch Einstellen der Blende 302 aus Fig. 3 im Beleuchtungsstrahlengang 111 für achsnahes Beleuchtungslicht kann die Helligkeit eines an einem Patientenauge hervorgerufenen roten Reflexes eingestellt werden. Die Blende 302 ist im Beleuchtungsstrahlengang 111 des Operationsmikroskops 100 so platziert, dass bei Schließen der Blende 302 das Leuchtfeld gleichmäßig dunkler wird ohne dass es dabei zu einseitigen Abschattungen kommt oder sich der Leuchtfelddurchmesser verringert.

Mittels der Blende 205 aus Fig. 2 im Strahlengang 112 für achsfernes Beleuchtungslicht kann dieser Strahlengang abgeschwächt und bei Bedarf vollständig unterbrochen werden.
Die Blende 205 ist dabei wiederum so im Strahlengang 112 platziert, dass bei Schließen das mit dem Strahlengang 112 im Objektbereich 105 des Operationsmikroskops hervorgerufene Leuchtfeld gleichmäßig dunkler wird, ohne dass es zu einseitigen Abschattungen kommt oder der Leuchtfelddurchmesser dabei verringert wird.

Die Beleuchtung des Objektbereichs ausschließlich mit achsnahem Beleuchtungslicht aus dem Beleuchtungsstrahlengang 111 aus Fig. 1 ist insbesondere bei Einsatz von Videodokumentationssystemen von Vorteil. Wenn kein achsfernes Beleuchtungslicht zum Objektbereich gelangt, wird bei einem Patientenauge vermieden, dass übermäßig viel Licht an dessen Skelera gestreut wird. Dies hat einen guten Bildkontrast zur Folge und es wird vermieden, dass aufgrund einer automatischen Einstellung von Belichtungszeit oder Verstärkung mittels einer Kamera erfasste Bilder eines Patientenauges im Bereich von dessen Pupille zu dunkel und zu kontrastarm sind.

Es versteht sich, dass für das Bewegen der verstellbaren Blenden im Operationsmikroskop auch elektrische Antriebe vorgesehen werden könnten. Auch ist es möglich, die Blenden für das Steuern mit getrennten Bedienelementen auszulegen.

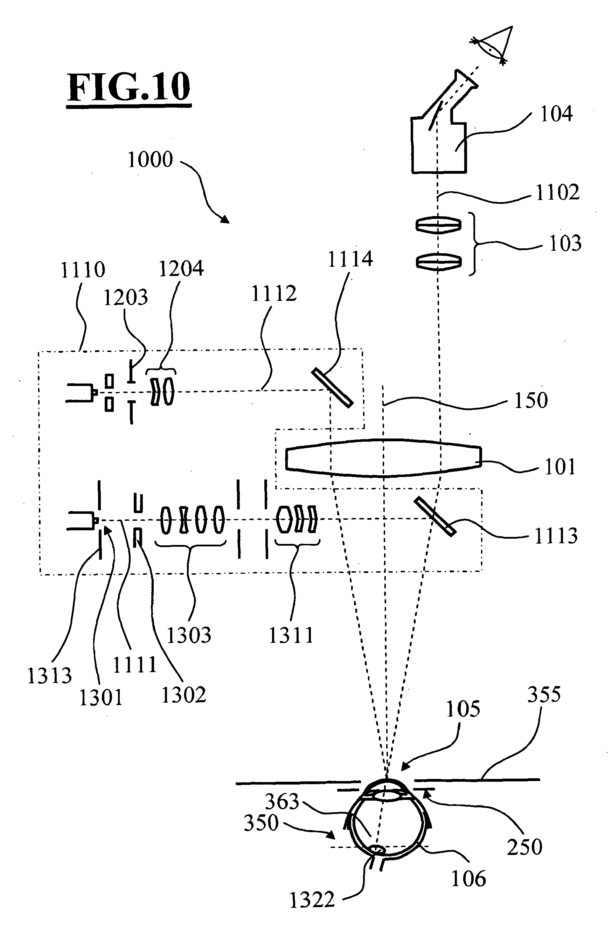

Die Fig. 10 zeigt ein weiteres Operationsmikroskop 1000. Soweit bei dem Operationsmikroskop 1000 Baugruppen vorgesehen sind, die den Baugruppen des Operationsmikroskops 100 aus Fig. 1 gleichen, sind diesen in Fig. 10 identische Bezugszeichen wie in Fig. 1 zugeordnet. Das Operationsmikroskop 1000 ist wiederum als Ophthalmo-Operationsmikroskop ausgelegt und eignet sich insbesondere zur Untersuchung eines in einem Objektbereich 105 angeordneten Patientenauges 106. Bei dem Operationsmikroskop 1000 in Fig. 10 liegt die Objektebene 355 im Bereich der Cornea 361 des Patientenauges 106, d.h. das Operationsmikroskop ist auf die Cornea des Patientenauges scharf gestellt.

Das Operationsmikroskop 1000 enthält eine Beleuchtungseinrichtung 1110, die in Bezug auf den stereoskopischen Beobachtungsstrahlengang 102 einen ersten Beleuchtungsstrahlengang 1111 für achsnahe Beleuchtung und einen zweiten Beleuchtungsstrahlengang 1112 für achsferne Beleuchtung des Objektbereichs bereitstellt.

Die Beleuchtungseinrichtung 1110 umfasst einen für Licht teildurchlässigen Spiegel 1113, der als erstes Umlenkelement wirkt, um dem stereoskopischen Beobachtungsstrahlengang 102 auf der Objekt zugeordneten Seite des Mikroskop-Hauptobjektivs 101 Beleuchtungslicht zu überlagern. Die Beleuchtungseinrichtung 1110 enthält einen Spiegel 1114 als zweites Umlenkelement, der achsfernes Beleuchtungslicht durch das Mikroskop-Hauptobjektiv 101 zum Objektbereich 105 lenkt.

Im Übrigen entspricht der prinzipielle Aufbau der Beleuchtungseinrichtung 1110 demjenigen der Beleuchtungseinrichtung 110 aus Fig. 1: Mittels einer Beleuchtungsoptik 1303, 1311 in der Beleuchtungseinrichtung 1110 wird eine Lichtaustrittseinheit 1301 in einer Lichtaustrittsebene 1313 im ersten Beleuchtungsstrahlengang 1111 durch eine einstellbare Aperturblende 1302 in eine erste Bildebene 350 abgebildet. Der zweite Beleuchtungsstrahlengang 1112 enthält eine Leuchtfeldblende 1203, die mit einer Beleuchtungsoptik 1204 durch das Mikroskop-Hauptobjektiv in eine von der ersten Bildebene 350 verschiedene zweite Bildebene 250 abgebildet wird. Die Beleuchtungsoptik 1302, 1303, 1311 ist dabei so ausgelegt, dass auf der Retina 363 des Patientenauges 106 ein Beleuchtungsspot 1322 erzeugt wird, dessen Durchmesser im Bereich 0,5 mm bis 1,5 mm liegt.

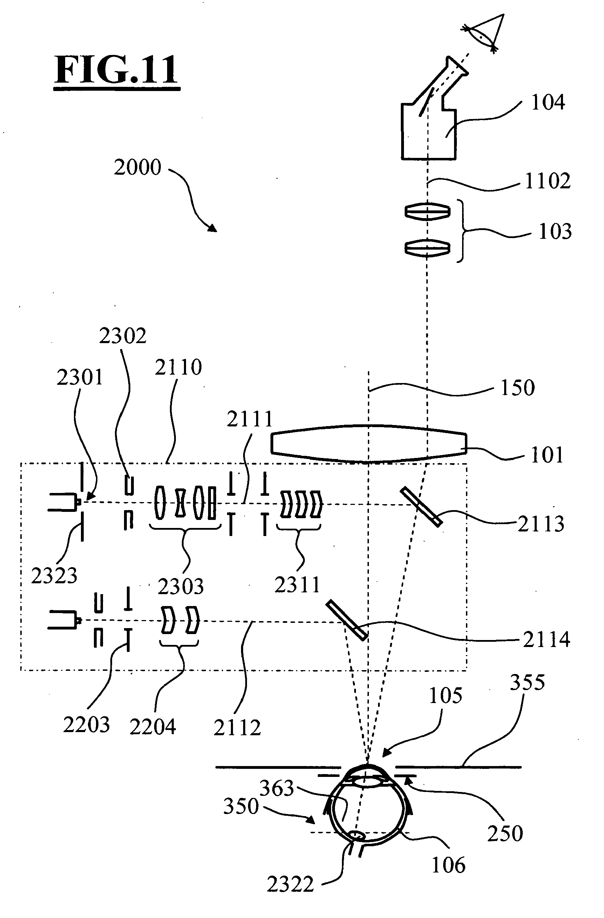

Ein weiteres Operationsmikroskop ist in Fig. 11 gezeigt. Das Operationsmikroskop 2000 in Fig. 11 hat entsprechend dem Operationsmikroskop 1000 aus Fig. 10 ein Mikroskop-Hauptobjektiv 101 mit einer optischen Achse 150, welches von einem stereoskopischen Beobachtungsstrahlengang 102 durchsetzt wird. Es ermöglicht einer Beobachtungsperson über ein Vergrößerungssystem 103 und einen Binokulartubus 104 einen Objektbereich 105 in einer Objektebene 355 zu untersuchen. Das Operationsmikroskop 2000 ist ebenfalls als Ophthalmo-Operationsmikroskop für die Untersuchung eines Patientenauges 106 ausgelegt.

Das Operationsmikroskop 2000 enthält eine Beleuchtungseinrichtung 2110, die in Bezug auf den stereoskopischen Beobachtungsstrahlengang 102 einen ersten Beleuchtungsstrahlengang 2111 für achsnahe Beleuchtung und einen zweiten Beleuchtungsstrahlengang 2112 für achs ferne Beleuchtung des Objektbereichs bereitstellt.

Die Beleuchtungseinrichtung 2110 umfasst einen für Licht teildurchlässigen Spiegel 2113, der als erstes Umlenkelement wirkt, um dem stereoskopischen Beobachtungsstrahlengang 102 auf der Objekt zugeordneten Seite des Mikroskop-Hauptobjektivs 101 Beleuchtungslicht zu überlagern. Die Beleuchtungseinrichtung 2110 enthält einen Spiegel 2114 als zweites Umlenkelement, der achsfernes Beleuchtungslicht am Mikroskop-Hauptobjektiv 101 vorbei zum Objektbereich 105 lenkt. Im Übrigen entspricht die Wirkungsweise der Beleuchtungseinrichtung 2110 derjenigen der Beleuchtungseinrichtung 110 aus Fig. 1 bzw. 1110 aus Fig. 10: Mittels einer Beleuchtungsoptik 2303, 2311 in der Beleuchtungseinrichtung 2110 wird eine Lichtaustrittseinheit 2301 mit einer Lichtaustrittsebene 2323 im ersten Beleuchtungsstrahlengang 2111 durch eine einstellbare Aperturblende 2302 in eine erste Bildebene 350 abgebildet. Der zweite Beleuchtungsstrahlengang 2112 enthält eine Leuchtfeldblende 2203, die mit einer Beleuchtungsoptik 2204 wiederum in eine von der ersten Bildebene 350 verschiedene zweite Bildebene 250 abgebildet wird. Die Beleuchtungsoptik 2302, 2303, 2311 im ersten Beleuchtungsstrahlengang ist dabei ebenfalls so ausgelegt, dass auf der Retina 363 des Patientenauges 106 ein Beleuchtungsspot 2322 erzeugt wird, dessen Durchmesser im Bereich 0,5 mm bis 1,5 mm liegt.

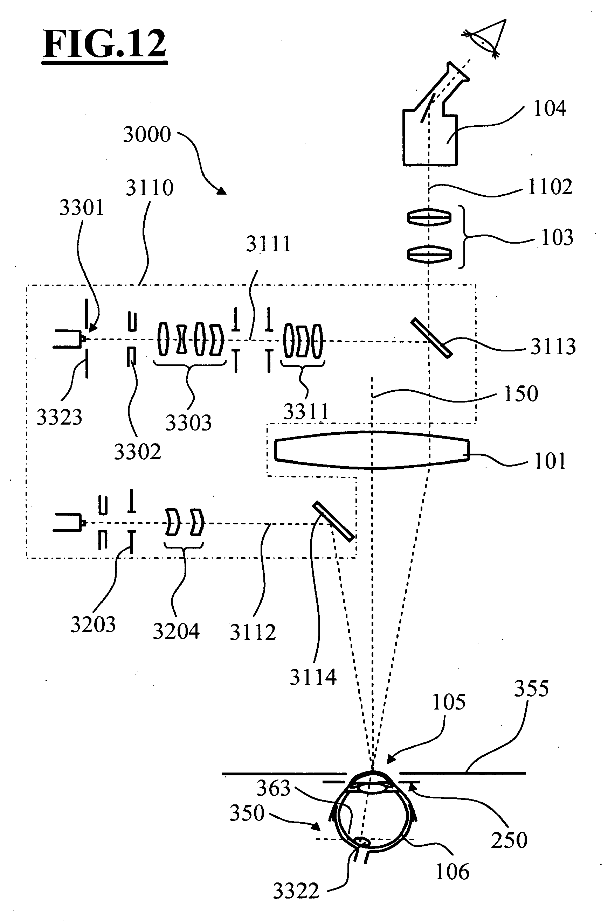

Die Fig. 12 zeigt ein Operationsmikroskop 3000. Das Operationsmikroskop 3000 hat ein Mikroskop-Hauptobjektiv 101 mit einer optischen Achse 150, welches von einem stereoskopischen Beobachtungsstrahlengang 102 durchsetzt wird. Es ermöglicht einer Beobachtungsperson über ein Vergrößerungssystem 103 und einen Binokulartubus 104 einen Objektbereich 105 in einer Objektebene 355 zu untersuchen. Das Operationsmikroskop 3000 ist ebenfalls als Ophthalmo-Operationsmikroskop ausgelegt und eignet sich so insbesondere zur Untersuchung eines Patientenauges 106.

Das Operationsmikroskop 3000 enthält wiederum wie die zuvor beschriebenen Operationsmikroskope eine Beleuchtungseinrichtung 3110, die in Bezug auf den stereoskopischen Beobachtungsstrahlengang 102 einen ersten Beleuchtungsstrahlengang 3111 für achsnahe Beleuchtung und einen zweiten Beleuchtungsstrahlengang 3112 für achs ferne Beleuchtung des Objektbereichs bereitstellt.

Die Beleuchtungseinrichtung 3110 umfasst einen für Licht teildurchlässigen Spiegel 3113, der als erstes Umlenkelement wirkt, um dem stereoskopischen Beobachtungsstrahlengang 102 durch das Mikroskop-Hauptobjektiv 101 hindurch, Beleuchtungslicht zu überlagern. Die Beleuchtungseinrichtung 3110 enthält einen Spiegel 3114 als zweites Umlenkelement, der achsfernes Beleuchtungslicht am das Mikroskop-Hauptobjektiv 101 vorbei zum Objektbereich 105 lenkt.

Wie bei dem Operationsmikroskop 100 aus Fig. 1, dem Operationsmikroskop 1000 aus Fig. 10 und dem Operationsmikroskop 2000 aus Fig. 11 wird bei dem Operationsmikroskop 3000 aus Fig. 12 eine Lichtaustrittseinheit 3301 mit einer Lichtaustrittsebene 3323 im ersten Beleuchtungsstrahlengang 3111 in eine erste Bildebene 350 abgebildet. Der zweite Beleuchtungsstrahlengang 3112 enthält eine Leuchtfeldblende 3203, die wiederum durch eine Beleuchtungsoptik 3204 in eine von der ersten Bildebene 350 verschiedene zweite Bildebene 250 abgebildet wird. Die Beleuchtungsoptik 3302, 3303, 3311 und 101 im ersten Beleuchtungsstrahlengang 3111 der Beleuchtungseinrichtung 3110 ist dabei so ausgelegt, dass auf der Retina 363 des Patientenauges 106 ein Beleuchtungsspot 3322 erzeugt wird, dessen Durchmesser im Bereich 0,5 mm bis 1,5 mm liegt.

Bei den erläuterten Operationsmikroskopen wird ein guter homogener und kontrastreicher Rotreflex an einem Patientenauge erzielt, indem die optische Achsen der Beobachtungsstrahlengänge des Operationsmikroskops am Hintergrund des Patientenauges nahe bei oder in einer optischen Achse des ersten Beleuchtungsstrahlengangs liegen.

Indem ein erster Beleuchtungsstrahlengang bereitgestellt wird, der optische Achsen hat, die am Augenhintergrund nahe der optischen Achse der Beobachtungsstrahlengänge oder in der optischen Achse der Beobachtungsstrahlengänge verlaufen, wird gewährleistet, dass im linken und rechten Beobachtungsstrahlengang des Operationsmikroskops ein gleichwertiger Rotreflex sichtbar ist.

Es sei bemerkt, dass es bei einem Operationsmikroskop mit mehreren stereoskopischen Beobachtungsstrahlengängen, etwa mit einem stereoskopischen Beobachtungsstrahlengang für Hauptbeobachtung und mit einem stereoskopischen Beobachtungsstrahlengang für Mitbeobachtung, günstig ist, einen ersten Beleuchtungsstrahlenganges vorzusehen, der Beleuchtungslicht bereitstellt, das mit mehreren optischen Achsen zum Objektbereich geführt wird, die den optischen Achsen der Beobachtungsstrahlengänge entsprechen. Auf diese Weise kann in sämtlichen Beobachtungsstrahlengängen eines Operationsmikroskops ein gutes, homogenes, kontrastreiches Bild des Vordergrunds eines Patientenauges unter Rotreflex sichtbar gemacht werden.

## Patentansprüche

1. Operationsmikroskop (100, 1000, 2000, 3000) mit einer Beleuchtungseinrichtung (110, 1110,2110,3110)
- bei dem die Beleuchtungseinrichtung (110, 1110, 2110, 3110) mit einem ersten Beleuchtungsstrahlengang (111, 1111, 2111, 3111) Beleuchtungslicht für den Objektbereich (105) und mit einem zweiten Beleuchtungsstrahlengang (112, 1112, 2112, 3112) Beleuchtungslicht für den Objektbereich (105) bereitstellen kann,
- wobei zum Bereitstellen von Licht in dem ersten Beleuchtungsstrahlengang (111, 1111, 2111, 3111) eine erste Lichtaustrittseinheit (301, 1301, 2301, 3301) vorgesehen ist; und
- in dem zweiten Beleuchtungsstrahlengang (112, 1112, 2112, 3112) eine Leuchtfeldblende (203, 1203, 2203, 3203) angeordnet ist,
- wobei der erste Beleuchtungsstrahlengang (111, 1111, 2111, 3111) eine Beleuchtungsoptik (302, 303, 306, 310, 311, 101, 1302, 1303, 1311, 2302, 2303, 2311, 3302, 3303, 3311, 101) aufweist, welche die Lichtaustritts-Ebene (313, 1313, 2323, 3332) der ersten Lichtaustrittseinheit (301, 1301, 2301, 3301) oder eine zur Lichtaustritts-Ebene (313, 1313, 2323, 3323) konjugierte Ebene in eine erste Bildebene (350) im unendlichen abbildet,
- wobei in dem zweiten Beleuchtungsstrahlengang (112, 1112, 2112, 3112) eine Beleuchtungsoptik (204, 101, 1204, 101, 2204, 3204) vorgesehen ist, welche die Leuchtfeldblende (203, 1203, 2203, 3203) in eine zweite, von der ersten Bildebene (350) verschiedene zweite Bildebene (250) abbildet.
- wobei der erste Beleuchtungsstrahlengang (111, 1111, 2111, 3111) relativ zu wenigstens einer optischen Achse (161) des Beobachtungsstrahlengang (102) des Operationsmikroskop mit einem achsnahen Verlauf zum Objektbereich (105) geführt ist,
- wobei der zweite Beleuchtungsstrahlengang (112, 1112, 2112, 3112) zu wenigstens einer optischen Achse (161) des Beobachtungsstrahlenganges (102) des Operationsmikroskops mit einem achsfernen Verlauf zum Objektbereich (105) geführt ist, und
- wobei die zweite Bildebene (250) in der das Bild der Leuchtfeldblende (203) im zweiten Beleuchtungsstrahlengang (112) liegt, in der Nähe der Objektebene (355) oder mit der Objektebene (355) des Operationsmikroskops (100) zusammenfällt.

2. Operationsmikroskop nach Anspruch 1 **dadurch gekennzeichnet, dass** der erste Beleuchtungsstrahlengang (111,3111) das Mikroskop-Hauptobj ektiv (101) durchsetzt

3. Operationsmikroskop nach Anspruch 2, **dadurch gekennzeichnet, dass** ein erstes Umlenkelement (113, 3113) vorgesehen ist, das Beleuchtungslicht durch das Mikroskop-Hauptobjektiv (101) zum Objektbereich (105) lenkt.

4. Operationsmikroskop nach Anspruch 3, **dadurch gekennzeichnet, dass** das erste Umlenkelement als teildurchlässiger Spiegel (113, 3113) ausgebildet ist, der von einem Beobachtungsstrahlengang (102) des Operationsmikroskops (100) durchsetzt wird.

5. Operationsmikroskop nach Anspruch 4, **dadurch gekennzeichnet, dass** der teildurchlässige Spiegel (113, 3113) als physikalischer Strahlteiler oder als geometrischer Strahlteiler ausgebildet ist.

6. Operationsmikroskop nach Anspruch 3, 4 oder 5, **dadurch gekennzeichnet, dass** das erste Umlenkelement (113, 3113) dazu dient, einem stereoskopischen Beobachtungsstrahlengang (102) Beleuchtungslicht koaxial zu überlagern.

7. Operationsmikroskop nach Anspruch 6, **dadurch gekennzeichnet, dass** das erste Umlenkelement (113, 3113) einem linken und einem rechten Beobachtungsstrahlengang (102) Beleuchtungslicht koaxial überlagert.

8. Operationsmikroskop nach einem der Ansprüche 2 bis 7, **dadurch gekennzeichnet, dass** ein zweites Umlenkelement (114, 1114) vorgesehen ist, das Beleuchtungslicht durch das Mikroskop-Hauptobjektiv (101) zum Objektbereich (105) lenkt.

9. Operationsmikroskop nach einem der Ansprüche 1 bis 8, **dadurch gekennzeichnet, dass** bei der Leuchtfeldblende (203) eine weitere Blende (202) angeordnet ist.

10. Operationsmikroskop nach Anspruch 9, **dadurch gekennzeichnet, dass** die Öffnung der weiteren Blende (202) variiert werden kann.

11. Operationsmikroskop nach Anspruch 9 oder 10, **dadurch gekennzeichnet, dass** die weitere Blende (202) als Retinaschutzblende ausgebildet ist.

12. Operationsmikroskop nach einem der Ansprüche 1 bis 11, **dadurch gekennzeichnet, dass** in dem zweiten Beleuchtungsstrahlengang (112) eine Aperturblende (205) angeordnet ist.

13. Operationsmikroskop nach Anspruch 12, **dadurch gekennzeichnet, dass** die Aperturblende (205) eine einstellbare Blendenöffnung hat.

14. Operationsmikroskop nach einem der Ansprüche 1 bis 13, **dadurch gekennzeichnet, dass** das in dem ersten Beleuchtungsstrahlengang (111) eine Blende (302) mit fester variierbarer, insbesondere Blendenöffnung ausgebildet ist, um die Lichtmenge in dem ersten Beleuchtungsstrahlengang (111) zu verändern.

15. Operationsmikroskop nach Anspruch 14, soweit Anspruch 14 auf Anspruch 13 und Anspruch 13 auf Anspruch 10 zurückbezogen ist, **dadurch gekennzeichnet, dass** zur Einstellung der Blende (202) in dem zweiten Beleuchtungsstrahlengang (112) und/oder der Aperturblende (205) in dem zweiten Beleuchtungsstrahlengang (112) und/oder der Blende (302) in dem ersten Beleuchtungsstrahlengang (111) ein gemeinsames benutzerbetätigbares Bedienelement (503) vorgesehen ist.

16. Operationsmikroskop nach Anspruch 15, **dadurch gekennzeichnet, dass** das benutzerbetätigbare Bedienelement als Drehknopf (503) ausgebildet ist.

17. Operationsmikroskop nach Anspruch 16, **dadurch gekennzeichnet, dass** der Drehknopf (503) mit einer Welleneinheit (506) gekoppelt ist, die über eine erste Steuerkurve (507) sowie einer ersten Abtriebseinheit (510) mit der Blende (302) in dem ersten Beleuchtungsstrahlengang (111) in Wirkverbindung steht, die über eine zweite Steuerkurve (508) sowie eine zweite Abtriebseinheit (511) mit der Blende (202) in dem zweiten Beleuchtungsstrahlengang' (112) in Wirkverbindung steht, und die über eine dritte Steuerkurve (509) sowie eine dritte Abtriebseinheit (512) mit der Aperturblende (205) Wirkverbindung steht.

18. Operationsmikroskop nach einem der Ansprüche 1 bis 17, **dadurch gekennzeichnet, dass** dem ersten Beleuchtungsstrahlengang (111) und dem zweiten Beleuchtungsstrahlengang (112) eine gemeinsame Lichtquelle (700) zugeordnet ist.

19. Operationsmikroskop nach Anspruch 18, **dadurch gekennzeichnet, dass** ein Lichtleiter (703) mit Verzweigung (704) vorgesehen ist, der das Licht der gemeinsamen Lichtquelle (700) dem ersten Beleuchtungsstrahlengang (111) und dem zweiten Beleuchtungsstrahlengang (112) zuführt.

20. Operationsmikroskop nach Anspruch 19, **dadurch gekennzeichnet, dass** der Lichtleiter (703) die erste Lichtaustrittseinheit (301) und eine zweite Lichtaustrittseinheit (201) aufweist.

21. Operationsmikroskop nach einem der Ansprüche 1 bis 20, **dadurch gekennzeichnet, dass** an der ersten Lichtaustrittseinheit (301) eine erste Beleuchtungspupille (314) und eine zweite Beleuchtungspupille (315) ausgebildet ist.

22. Operationsmikroskop nach Anspruch 20 oder Anspruch 21, **dadurch gekennzeichnet, dass** an der zweiten Lichtaustrittseinheit (201) eine einzige Beleuchtungspupille (901) ausgebildet ist.

23. Operationsmikroskop nach einem der Ansprüche 1 bis 22, **dadurch gekennzeichnet, dass** in dem ersten Beleuchtungsstrahlengang (111) eine Reflexblende (324) vorgesehen ist, die Beleuchtungsstrahlen abschattet, welche durch Reflexionen an optischen Elementen im Operationsmikroskop, störendes Streulicht in den Beobachtungsstrahlengängen des Operationsmikroskops (100) hervorrufen.

24. Operationsmikroskop nach Anspruch 23, **dadurch gekennzeichnet, dass** die Reflexblende (324) derart angeordnet ist, dass keine Beobachtungsstrahlen im Operationsmikroskop ausgeblendet werden und kein Beschnitt des Bildes der Leuchtfeldblende (203) in der zweiten Bildebene (250) durch die Reflexblende (324) erfolgt.

25. Operationsmikroskop nach einem der Ansprüche 1 bis 24 **dadurch gekennzeichnet, dass** die Beleuchtungseinrichtung (110, 1110, 2110, 3110) derart ausgelegt ist, dass auf der Retina (363) eines idealen Patientenauges (106) mit Beleuchtungslicht aus dem ersten Beleuchtungsstrahlengang (111, 1111, 2111, 3111) ein Beleuchtungsspot (322, 323) erzeugt werden kann, dessen Durchmesser im Bereich 0,5 mm bis 1,5 mm liegt.

26. Operationsmikroskop nach Anspruch 25, **dadurch gekennzeichnet, dass** die Beleuchtungseinrichtung (110, 1110, 2110, 3110) ausgelegt ist, dass auf der Retina (363) eines idealen Patientenauges (106) mit Beleuchtungslicht aus dem ersten Beleuchtungsstrahlengang (111, 1111, 2111, 3111) zwei Beleuchtungsspots erzeugt werden können, deren Durchmesser jeweils im Bereich 0,5 mm bis 1,5 mm liegt.

## Claims

1. Surgical microscope (100, 1000, 2000, 3000) with an illumination device (110, 1110, 2110, 3110)
- in which the illumination device (110, 1110, 2110, 3110) can provide illumination light for the object region (105) with a first illumination beam path (111, 1111, 2111, 3111) and can provide illumination light for the object region (105) with a second illumination beam path (112, 1112, 2112, 3112),
- wherein provision is made for a first light-outlet unit (301, 1301, 2301, 3301) for providing light in the first illumination beam path (111, 1111, 2111, 3111); and
- a field stop (203, 1203, 2203, 3203) is arranged in the second illumination beam path (112, 1112, 2112, 3112),
- wherein the first illumination beam path (111, 1111, 2111, 3111) has an illumination optical system (302, 303, 306, 310, 311, 101, 1302, 1303, 1311, 2302, 2303, 2311, 3302, 3303, 3311, 101), which images the light-outlet plane (313, 1313, 2323, 3323) of the first light-outlet unit (301, 1301, 2301, 3301), or a conjugate plane to the light-outlet plane (313, 1313, 2323, 3323), at infinity in a first image plane (350),
- wherein an illumination optical system (204, 101, 1204, 101, 2204, 3204) is provided in the second illumination beam path (112, 1112, 2112, 3112), which illumination optical system images the field stop (203, 1203, 2203, 3203) in a second image plane (250), which differs from the first image plane (350),
- wherein, relative to at least one optical axis (161) of the observation beam path (102) of the surgical microscope, the first illumination beam path (111, 1111, 2111, 3111) is guided to the object region (105) such that it runs close to said axis,
- wherein the second illumination beam path (112, 1112, 2112, 3112) is guided to the object region (105) such that it runs away from the axis with respect to at least one optical axis (161) of the observation beam path (102) of the surgical microscope, and
- wherein the second image plane (250), in which the image of the field stop (203) lies in the second illumination beam path (112), lies in the vicinity of the object plane (355) or coincides with the object plane (355) of the surgical microscope (100).

2. Surgical microscope according to Claim 1, **characterized in that** the first illumination beam path (111, 3111) passes through the main microscope objective (101).

3. Surgical microscope according to Claim 2, **characterized in that** provision is made for a first deflection element (113, 3113), which guides illumination light to the object region (105) through the main microscope objective (101).

4. Surgical microscope according to Claim 3, **characterized in that** the first deflection element is designed as a partly transmitting mirror (113, 3113), through which an observation beam path (102) of the surgical microscope (100) passes.

5. Surgical microscope according to Claim 4, **characterized in that** the partly transmitting mirror (113, 3113) is designed as a physical beamsplitter or as a geometric beamsplitter.

6. Surgical microscope according to Claim 3, 4, or 5, **characterized in that** the first deflection element (113, 3113) is used to superpose illumination light in a coaxial fashion onto a stereoscopic observation beam path (102).

7. Surgical microscope according to Claim 6, **characterized in that** the first deflection element (113, 3113) coaxially superposes illumination light onto a left and a right illumination beam path (102).

8. Surgical microscope according to one of Claims 2 to 7, **characterized in that** provision is made for a second deflection element (114, 1114), which guides illumination light to the object region (105) through the main microscope objective (101).

9. Surgical microscope according to one of Claims 1 to 8, **characterized in that** an additional stop (202) is arranged at the field stop (203).

10. Surgical microscope according to Claim 9, **characterized in that** the opening of the additional stop (202) can be varied.

11. Surgical microscope according to Claim 9 or 10, **characterized in that** the additional stop (202) is designed as a retina protection stop.

12. Surgical microscope according to one of Claims 1 to 11, **characterized in that** an aperture stop (205) is arranged in the second illumination beam path (112).

13. Surgical microscope according to Claim 12, **characterized in that** the aperture stop (205) has an adjustable aperture.

14. Surgical microscope according to one of Claims 1 to 13, **characterized in that** a stop (302) with a fixed variable aperture for changing the amount of light in the first illumination beam path (111) is formed in the first illumination beam path (111).

15. Surgical microscope according to Claim 14, provided Claim 14 refers back to Claim 13 and Claim 13 refers back to Claim 12, **characterized in that** provision is made for a common operating element (503) that can be actuated by the user for adjusting the stop (202) in the second illumination beam path (112) and/or the aperture stop (205) in the second illumination beam path (112) and/or the stop (302) in the first illumination beam path (111).

16. Surgical microscope according to Claim 15, **characterized in that** the operating element that can be actuated by the user is designed as a knob (503).

17. Surgical microscope according to Claim 16, **characterized in that** the knob (503) is coupled to a shaft unit (506), which is operatively connected to the stop (302) in the first illumination beam path (111) via a first radial cam (507) and a first driven unit (510), which shaft unit is operatively connected to the stop (202) in the second illumination beam path (112) via a second radial cam (508) and a second driven unit (511), and which shaft unit is operatively connected to the aperture stop (205) via a third radial cam (509) and a third driven unit (512).

18. Surgical microscope according to one of Claims 1 to 17, **characterized in that** a common light source (700) is assigned to the first illumination beam path (111) and the second illumination beam path (112).

19. Surgical microscope according to Claim 18, **characterized in that** provision is made for an optical waveguide (703) with a branching (704), which optical waveguide feeds the light from the common light source (700) to the first illumination beam path (111) and the second illumination beam path (112).

20. Surgical microscope according to Claim 19, **characterized in that** the optical waveguide (703) has the first light-outlet unit (301) and a second light-outlet unit (201).

21. Surgical microscope according to one of Claims 1 to 20, **characterized in that** a first illumination pupil (314) and a second illumination pupil (315) are formed on the first light-outlet unit (301).

22. Surgical microscope according to Claim 20 or Claim 21, **characterized in that** a single illumination pupil (901) is formed on the second light-outlet unit (201).

23. Surgical microscope according to one of Claims 1 to 22, **characterized in that** provision is made for a reflection stop (324) in the first illumination beam path (111), the former shadowing illumination beams that cause distracting stray light in the observation beam paths of the surgical microscope (100), which stray light is the result of reflections on optical elements in the surgical microscope.

24. Surgical microscope according to Claim 23, **characterized in that** the reflection stop (324) is arranged such that no observation beams are masked in the surgical microscope and no image of the field stop (203) is trimmed in the second image plane (250) by the reflection stop (324).

25. Surgical microscope according to one of Claims 1 to 24, **characterized in that** the illumination device (110, 1110, 2110, 3110) is designed such that an illumination spot (322, 323) with a diameter in the region of 0.5 mm to 1.5 mm can be generated on the retina (363) of an ideal patient eye (106) by illumination light from the first illumination beam path (111, 1111, 2111, 3111).

26. Surgical microscope according to Claim 25, **characterized in that** the illumination device (110, 1110, 2110, 3110) is designed such that two illumination spots, each with a diameter in the region of 0.5 mm to 1.5 mm, can be generated on the retina (363) of an ideal patient eye (106) by illumination light from the first illumination beam path (111, 1111, 2111, 3111).

## Revendications

1. Microscope d'opération (100, 1000, 2000, 3000) doté d'un dispositif d'éclairage (110, 1110, 2110, 3110)
- dans lequel le dispositif d'éclairage (110, 1110, 2110, 3110) peut fournir de la lumière d'éclairage pour l'espace objet (105) au moyen d'un premier chemin de faisceau d'éclairage (111, 1111, 2111, 3111) et de la lumière d'éclairage pour l'espace objet au moyen d'un deuxième chemin de faisceau d'éclairage (112, 1112, 2112, 3112),
- une première unité de sortie de lumière (301, 1301, 2301, 3301) étant prévue pour fournir de la lumière au premier chemin de faisceau d'éclairage (111, 1111, 2111, 3111) ; et
- un diaphragme du champ d'éclairage (203, 1203, 2203, 3203) étant disposé sur le deuxième chemin de faisceau d'éclairage (112, 1112, 2112, 3112),
- le premier chemin de faisceau d'éclairage (111, 1111, 2111, 3111) comportant une optique d'éclairage (302, 303, 306, 310, 311, 101, 1302, 1303, 1311, 2302, 2303, 2311, 3302, 3303, 3311, 101) qui reproduit dans un premier plan image (350) à l'infini, le plan de sortie de la lumière (313, 1313, 2323, 3323) de la première unité de sortie de lumière (301, 1301, 2301, 3301) ou un plan conjugué du plan de sortie de la lumière (313, 1313, 2323, 3323),
- une optique d'éclairage (204, 101, 1204, 101, 2204, 3204) étant prévue sur le deuxième chemin de faisceau d'éclairage (112, 1112, 2112, 3112) qui reproduit le diaphragme du champ d'éclairage (203, 1203, 2203, 3203) dans un deuxième plan image (250) différent du premier plan image (350),
- le premier chemin de faisceau d'éclairage (111, 1111, 2111, 3111) étant acheminé vers l'espace objet (105) selon un tracé proche de l'axe par rapport à au moins un axe optique (161) du chemin de faisceau d'observation (102) du microscope d'opération,
- le deuxième chemin de faisceau d'éclairage (112, 1112, 2112, 3112) étant acheminé vers l'espace objet (105) selon un tracé éloigné de l'axe par rapport à au moins un axe optique (161) du chemin de faisceau d'observation (102) du microscope d'opération,
- le deuxième plan image (250), dans lequel se trouve l'image du diaphragme du champ d'éclairage (203) sur le deuxième chemin de faisceau d'éclairage (112), se trouvant dans la proximité du plan objet (355) ou coïncidant avec le plan objet (355) du microscope d'opération (100).

2. Microscope d'opération selon la revendication 1, **caractérisé en ce que** le premier chemin de faisceau d'éclairage (111, 3111) traverse l'objectif principal (101) du microscope.

3. Microscope d'opération selon la revendication 2, **caractérisé en ce qu'**un premier élément de déviation (113, 3113) est prévu pour diriger la lumière d'éclairage à travers l'objectif principal (101) du microscope vers l'espace objet (105).

4. Microscope d'opération selon la revendication 3, **caractérisé en ce que** le premier élément de déviation (113, 3113) est réalisé sous la forme d'un miroir semi-transparent traversé par un chemin de faisceau d'observation (102) du microscope d'opération (100).

5. Microscope d'opération selon la revendication 4, **caractérisé en ce que** le miroir semi-transparent (113, 3113) est réalisé sous la forme d'un séparateur de faisceau physique ou d'un séparateur de faisceau géométrique.

6. Microscope d'opération selon la revendication 3, 4 ou 5, **caractérisé en ce que** le premier élément de déviation (113, 3113) sert à superposer de manière coaxiale de la lumière d'éclairage à un chemin de faisceau d'observation stéréoscopique (102).

7. Microscope d'opération selon la revendication 6, **caractérisé en ce que** le premier élément de déviation (113, 3113) superpose de manière coaxiale de la lumière d'éclairage à un chemin de faisceau d'observation gauche et à un chemin de faisceau d'observation droit (102).

8. Microscope d'opération selon une des revendications 2 à 7, **caractérisé en ce qu'**un deuxième élément de déviation (114, 1114) est prévu pour diriger la lumière d'éclairage à travers l'objectif principal (101) du microscope vers l'espace objet (105).

9. Microscope d'opération selon une des revendications 1 à 8, **caractérisé en ce qu'**un autre diaphragme (202) est disposé près du diaphragme du champ d'éclairage (203).

10. Microscope d'opération selon la revendication 9, **caractérisé en ce qu'**on peut modifier l'ouverture de l'autre diaphragme (202).

11. Microscope d'opération selon la revendication 9 ou 10, **caractérisé en ce que** l'autre diaphragme (202) est réalisé comme diaphragme de protection de la rétine.

12. Microscope d'opération selon une des revendications 1 à 11, **caractérisé en ce qu'**un diaphragme d'ouverture (205) est disposé sur le deuxième chemin de faisceau d'éclairage (112).

13. Microscope d'opération selon la revendication 12, **caractérisé en ce que** le diaphragme d'ouverture (205) dispose d'une ouverture de diaphragme réglable.

14. Microscope d'opération selon une des revendications 1 à 13, **caractérisé en ce qu'**un diaphragme (302) est réalisé sur le premier chemin de faisceau d'éclairage (111) avec une ouverture de diaphragme fixe réglable pour modifier la quantité de lumière sur le premier chemin de faisceau d'éclairage (111).

15. Microscope d'opération selon la revendication 14, dans le mesure où la revendication 14 se réfère à la revendication 13 et la revendication 13 se réfère à la revendication 12, **caractérisé en ce qu'**un élément de manoeuvre commun (503) pouvant être actionné par l'utilisateur est prévu pour le réglage du diaphragme (202) sur le deuxième chemin de faisceau d'éclairage (112) et/ou du diaphragme d'ouverture (205) sur le deuxième chemin de faisceau d'éclairage (112) et/ou du diaphragme (302) sur le premier chemin de faisceau d'éclairage (111).

16. Microscope d'opération selon la revendication 15, **caractérisé en ce que** l'élément de manoeuvre pouvant être actionné par l'utilisateur est réalisé sous forme de bouton rotatif (503).

17. Microscope d'opération selon la revendication 16, **caractérisé en ce que** le bouton rotatif (503) est accouplé à une unité d'arbre (506) se trouvant en liaison active avec le diaphragme (302) sur le premier chemin de faisceau d'éclairage (111) par l'intermédiaire d'une première came (507) ainsi que d'une première unité d'entraînement (510), qui se trouve en liaison active avec le diaphragme (202) sur le deuxième chemin de faisceau d'éclairage (112) par l'intermédiaire d'une deuxième came (508) ainsi que d'une deuxième unité d'entraînement (511) et qui se trouve en liaison active avec le diaphragme d'ouverture (205) par l'intermédiaire d'une troisième came (509) ainsi que d'une troisième unité d'entraînement (512).

18. Microscope d'opération selon une des revendications 1 à 17, **caractérisé en ce qu'**une source lumineuse commune (700) est attribuée au premier chemin de faisceau d'éclairage (111) et au deuxième chemin de faisceau d'éclairage (112).

19. Microscope d'opération selon la revendication 18, **caractérisé en ce qu'**un conduit de lumière (703) avec bifurcation (704) est prévu pour acheminer la lumière de la source lumineuse commune (700) vers le premier chemin de faisceau d'éclairage (111) et le deuxième chemin de faisceau d'éclairage (112).

20. Microscope d'opération selon la revendication 19, **caractérisé en ce que** le conduit de lumière (703) comporte la première unité de sortie de lumière (301) et une deuxième unité de sortie de lumière (201).

21. Microscope d'opération selon une des revendications 1 à 20, **caractérisé en ce qu'**une première pupille d'éclairage (314) et une deuxième pupille d'éclairage (315) sont réalisées dans la première unité de sortie de lumière (301).

22. Microscope d'opération selon la revendication 20 ou la revendication 21, **caractérisé en ce qu'**une seule pupille d'éclairage (901) est réalisée dans la deuxième unité de sortie de lumière (201).

23. Microscope d'opération selon une des revendications 1 à 22, **caractérisé en ce qu'**un diaphragme reflex (324) est prévu sur le premier chemin de faisceau d'éclairage (111) qui éclipse les rayons d'éclairage générant par réflexion sur les éléments optiques dans le microscope d'opération, de la lumière diffuse dans les chemins de faisceaux d'observation du microscope d'opération (100).

24. Microscope d'opération selon la revendication 23, **caractérisé en ce que** le diaphragme reflex (324) est disposé de telle sorte qu'aucun rayon d'observation n'est occulté dans le microscope d'opération et que le diaphragme reflex (324) n'engendre aucune coupure de l'image du diaphragme du champ d'éclairage (203) dans le deuxième plan image (250).

25. Microscope d'opération selon une des revendications 1 à 24, **caractérisé en ce que** le dispositif d'éclairage (110, 1110, 2110, 3110) est réalisé de telle sorte qu'une tache d'éclairage (322, 323) d'un diamètre situé dans la plage de 0,5 mm à 1,5 mm puisse être générée par la lumière d'éclairage provenant du premier chemin de faisceau d'éclairage (111, 1111, 2111, 3111) sur la rétine (363) d'un oeil idéal (106) de patient.

26. Microscope d'opération selon la revendication 25, **caractérisé en ce que** le dispositif d'éclairage (110, 1110, 2110, 3110) est réalisé de telle sorte que deux taches d'éclairage chacune d'un diamètre situé dans la plage de 0,5 mm à 1,5 mm puissent être générées par la lumière d'éclairage provenant du premier chemin de faisceau d'éclairage (111, 1111, 2111, 3111) sur la rétine (363) d'un oeil idéal (106) de patient.
